(19) Europäisches Patentamt
European Patent Office
Office européen des brevets

(11) EP 1 048 289 A2

(12) # EUROPÄISCHE PATENTANMELDUNG

(43) Veröffentlichungstag:
**02.11.2000 Patentblatt 2000/44**

(51) Int. Cl.[7]: **A61K 7/13**

(21) Anmeldenummer: **00108529.9**

(22) Anmeldetag: **19.04.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **27.04.1999 DE 19919089**

(71) Anmelder:
**Cognis Deutschland GmbH**
**40589 Düsseldorf (DE)**

(72) Erfinder:
- **Seipel, Werner**
  **40723 Hilden (DE)**
- **Kleen, Astrid, Dr.**
  **40699 Erkrath (DE)**

(54) **Haarfärbepräparate**

(57) Vorgeschlagen werden Haarfärbepräparate, enthaltend

(a) 0,5 bis 25 Gew.-% Fettsäure(polyglycol)ester,
(b) 0,5 bis 25 Gew.-% Fettsäurepartialglyceride und
(c) 0,1 bis 15 Gew.-% Haarfarbstoffe

mit der Maßgabe, daß sich die Mengenangaben mit Wasser und gegebenenfalls weiteren Hilfs- und Zusatzstoffen zu 100 Gew.-% ergänzen.



Printed by Xerox (UK) Business Services
2.16.7 (HRS)/3.6

**Beschreibung**

**Gebiet der Erfindung**

Die Erfindung betrifft Haarfärbepräparate, die Fettsäure(polyglycol)ester, Fettsäurepartialglyceride und Haarfarbstoffe enthalten, sowie deren Verwendung als Konsistenzgeber zur Herstellung von Haarfärbepräparaten.

**Stand der Technik**

Zubereitungen, die zur Reinigung und Pflege der Haare eingesetzt werden, enthalten in der Regel ein oder mehrere oberflächenaktive Substanzen, insbesondere auf Basis von anionischen oder amphoteren Tensiden. Die alleinige Anwendung von Tensiden in Haarfärbepräparaten, wie z.B. Cremes vom Öl-in-Wasser-Typ, würde jedoch zum Austrocknen der Haare führen, so daß in der Regel pflegende Substanzen zugesetzt werden. Nach wie vor besteht allerdings das Bedürfnis Haarfärbepräparate zur Verfügung zu stellen, die einen noch stärkeren Schutz der Haare beim Färben liefern und weiterhin der Schädigung der Haarstruktur durch Färbevorgänge bei guter Hautverträglichkeit entgegenwirken. Diese Präparate sollen sich zusätzlich durch eine gute Stabilität auch bei Temperaturlagerung auszeichnen.

Die Aufgabe der Erfindung hat folglich darin bestanden, Haarfärbepräparate zur Verfügung zu stellen, die nach der Anwendung die Haare kräftigen und stabilisieren und somit eine Verbesserung der Haarstruktur bewirken. Diese Mischungen sollen ebenfalls eine gute dermatologische Verträglichkeit aufweisen und sich durch eine gute Stabilität bei Temperaturlagerung auszeichnen.

**Beschreibung der Erfindung**

Gegenstand der Erfindung sind Haarfärbepräparate, enthaltend

(a) 0,5 bis 25 Gew.-% Fettsäure(polyglycol)ester,
(b) 0,5 bis 25 Gew.-% Fettsäurepartialglyceride und
(c) 0,1 bis 15 Gew.-% Haarfarbstoffe

mit der Maßgabe, daß sich die Mengenangaben mit Wasser und gegebenenfalls weiteren Hilfs- und Zusatzstoffen zu 100 Gew.-% ergänzen.

Überraschenderweise wurde gefunden, daß Haarfärbepräparate, vorzugsweise O/W-Cremezubereitungen, welche eine Mischung aus Fettsäure(polyglycol)estern, ausgewählten Partialglyceriden und Färbepräparaten enthalten, zu einer Verbesserung der Haarstruktur und somit zu eine Stabilisierung und Kräftigung der Haare führen. Die Erfindung schließt die Erkenntnis mit ein, daß diese Mittel einen gleichzeitigen Schutz der Haare vor Austrocknung und Feuchtigkeitsverlust liefern und somit zu deren Schonung beitragen. Darüber hinaus zeigen diese Mischungen einen rückfettenden Effekt und gute dermatologische Verträglichkeit. Die resultierenden Haarfärbepräparate zeichnen sich hierbei durch eine besonders hohe Stabilität auch bei Temperaturlagerung aus.

<u>Fettsäure(polyglycol)estersulfate</u>

Die erfindungsgemäßen Mittel können Fettsäure(polyglycol)estersulfate, welche die Komponente (a) bilden, der Formel **(I)** enthalten,

$$R^1COO(AO)_xR^2 \qquad (I)$$

in der $R^1CO$ für einen linearen oder verzweigten, gesättigten oder ungesättigten Acylrest mit 6 bis 30, vorzugsweise 8 bis 22 und insbesondere 10 bis 18 Kohlenstoffatomen, x für Zahlen von durchschnittlich 0 bis 30, vorzugsweise 5 bis 20 und insbesondere 10 bis 15 steht und AO für einen $CH_2CH_2O$-, $CH_2CH(CH_3)O$- und/oder $CH(CH_3)CH_2O$-Rest und $R^2$ für einen linearen oder verzweigten, gesättigten oder ungesättigten Alkylrest mit 1 bis 4 und vorzugsweise 1 und/oder 2 Kohlenstoffatomen und insbesondere Methyl steht, werden durch Veresterung der entsprechenden Fettsäure(polyglycole) hergestellt. Diese wiederum sind nach den einschlägigen präparativen Verfahren der organischen Chemie erhältlich. Hierzu wird Ethylenoxid, Propylenoxid oder deren Gemisch - in random - oder Blockverteilung - gegebenenfalls an die entsprechenden Fettsäuren angelagert, wobei diese Reaktion säurekatalysiert, vorzugsweise

aber in Gegenwart von Basen, wie z.B. Natriummethylat oder calciniertem Hydrotalcit erfolgt. Typische Beispiele für geeignete Ausgangsstoffe sind die Anlagerungsprodukte von 10 bis 15 Mol Ethylenoxid und/oder Propylenoxid, vorzugsweise aber die Addukte mit 12 Mol Ethylenoxid oder 12 Mol Propylenoxid an Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die dann verestert werden.

<u>Fettsäurepartialglyceride</u>

Fettsäurepartialglyceride, welche die Komponente (b) bilden, also Monoglyceride, Diglyceride und deren technische Gemische, können herstellungsbedingt noch geringe Mengen Triglyceride enthalten. Die Partialglyceride folgen vorzugsweise der Formel **(II)**,

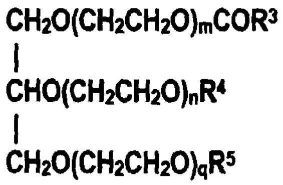

**(II)**

in der $R^3CO$ für einen linearen oder verzweigten, gesättigten und/oder ungesättigten Acylrest mit 6 bis 22, vorzugsweise 12 bis 18 Kohlenstoffatomen, $R^4$ und $R^5$ unabhängig voneinander für $R^3CO$ oder OH und die Summe ( m+n+q ) für 0 oder Zahlen von 1 bis 100, vorzugsweise 5 bis 25 steht, mit der Maßgabe, daß mindestens einer der beiden Reste $R^4$ und $R^5$ OH bedeutet. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

<u>Haarfarbstoffe</u>

Als Haarfarbstoffe, welche die Komponente (c) bilden, kommen beispielsweise **direktziehende Farbstoffe**, z.B. aus der Gruppe der Nitrophenylendiamine, Nitroaminophenole, Anthrachinone oder Indophenole in Betracht, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'-carbonsäure, 6-Nitro-1,2,3,4-tetrahydrochinoxalin, (N-2,3-Dihydroxypropyl-2-nitro-4-trifluormethyl)-ami-nobenzol und 4-N-Ethyl-1,4-bis(2'-hydroxyethylamino)-2-nitro-benzolhydrochlorid. Weiterhin können den Emulsionen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel zugesetzt werden.

Neben den Direktziehern können den Emulsionen auch **Oxidationsfarbstoffe**, bestehend aus Entwickler- und Kupplerkomponente zugesetzt werden. Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind u.a. p-Toluylendiamin, p-Aminophenol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5 und 4-Amino-3-methylphenol, 2-(2-Hydroxyethyl)-1,4-aminobenzol und 2,4,5,6-Tetraaminopyrimidin. Als Kupplerkomponenten werden in der Regel m-Phenylendiaminderivate, Naphthole, Resorcin und Resorcinderivate, Pyrazolone, m-Aminophenole sowie Pyridin-Derivate verwendet. Als Kupplersubstanzen eignen sich insbesondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 2,5-Dimethylresorcin, 2,6-Dihydroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen. Eine Übersicht zu geeigneten Farbstoffen ist weiterhin der Publikation **"Kosmetische Färbemittel" der Farbstoffkommission der Deutschen Forschungsge-**

**meinschaft, Verlag Chemie, Weinheim, 1984, S.81-106** zu entnehmen.

Tenside

Die erfindungsgemäßen Mittel können als bevorzugte Hilfs- und Zusatzstoffe anionische und/oder amphotere bzw. zwitterionische Tenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Der Anteil der Tenside an den Mitteln kann 0,1 bis 10 und vorzugsweise 0,5 bis 5 Gew.-% betragen.

Ölkomponenten

Die erfindungsgemäßen Mittel können bevorzugte Hilfs- und Zusatzstoffe 0,5 bis 25 und vorzugsweise 1 bis 15 Gew.-% Ölkörper enthalten. Als Ölkörper beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von Hydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht. Als Ölkörper können desweiteren Kohlenwasserstoffe wie Squalan und Squalen eingesetzt werden.

Polymere Verdickungsmittel

In einer weiteren bevorzugten Ausführungsform der Erfindung enthalten die erfindungsgemäßen Haarfärbepräparate weiterhin als Hilfs- und Zusatzstoffe polymere Verdickungsmittel, wie Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid. Die Polymeren können in Mengen von 0,1 bis 5, vorzugsweise 0,5 bis 2 Gew.-% - bezogen auf die Haarfärbepräparate - eingesetzt werden.

Fettalkohole

Die erfindungsgemäßen Mittel können weiterhin als bevorzugte Hilfs- und Zusatzstoffe primäre aliphatische Fettalkohole der Formel **(III)** enthalten,

$$R^6OH \quad \text{(III)}$$

in der $R^6$ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelenschen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol. Der Anteil der Fettalkohole in den Mitteln kann dabei 5 bis 50 und vorzugsweise 25 bis 40 Gew.-% betragen

Die erfindungsgemäßen Mittel können mit Hilfe des Mikrodispersionsverfahren als Fettalkoholdispersionen hergestellt werden. Mikrodispersionen stellen optisch isotrope, thermodynamisch stabile Systeme dar, die eine wasserunlösliche Ölkomponente (hier: Fettalkoholpartialglyceride), Dispergatoren - vorzugsweise Alkylglucoside - und Wasser enthalten. Das klare bzw. transparente Aussehen der Mikrodispersionen ist eine Folge der geringen Teilchengröße der dispergierten Emulsionströpfchen. In diesem Zusammenhang hat sich erwiesen, daß Fettalkoholmikrodispersionen einen besonders vorteilhaften Einfluß auf die Herstellung und Lagerstabilität der resultierenden Haarfärbepräparate besitzen. Vorzugsweise werden Dispersionen eingesetzt, die eine Teilchengröße kleiner 50 µm, insbesondere kleiner 20 µm und besonders bevorzugt kleiner 10 µm aufweisen. Übersichten zu Herstellung und Anwendung von Mikrodispersionen finden sich von H. Eicke in **SÖFW-Journal, <u>118</u>, 311 (1992)** und Th. Förster et al. in **SÖFW-Journal, <u>122</u>, 746 (1996)**; des weiteren sei auf die Druckschriften **DE 4411557 A1** (Henkel) und **EP 0687206 A1** (L'Oréal) verwiesen.

**Gewerbliche Anwendbarkeit**

Ein weiterer Gegenstand der Erfindung betrifft die Verwendungen von Mitteln, enthaltend

(a) 0,5 bis 25, vorzugsweise 5 bis 20 und insbesondere 10 bis 15 Gew.-% Fettsäure(polyglycol)ester,
(b) 0,5 bis 25, vorzugsweise 5 bis 20 und insbesondere 10 bis 15 Gew.-% Fettsäurepartialglyceride und
(c) 0,1 bis 15, vorzugsweise 0,5 bis 10 und insbesondere 1 bis 5 Gew.-% Haarfarbstoffe

mit der Maßgabe, daß sich die Mengenangaben mit Wasser und gegebenenfalls weiteren Hilfs- und Zusatzstoffen zu 100 Gew.-% ergänzen, als Konsistenzgeber zur Herstellung von Haarfärbepräparaten, vorzugsweise von O/W-Färbecremes.

Hilfs- und Zusatzstoffe

Bei den erfindungsgemäßen Haarfärbepräparaten handelt es sich vorzugsweise um O/W-Cremes, die als zusätzliche Hilfs- und Zusatzstoffe Co-Emulgatoren, Überfettungsmittel, Perlglanzwachse, Konsistenzgeber, Polymere, Siliconverbindungen, Fette, Wachse, Stabilisatoren, biogene Wirkstoffe, Antischuppenmittel, Filmbildner, Quellmittel, UV-Lichtschutzfaktoren, Hydrotrope, Konservierungsmittel, Solubilisatoren, Komplexbildner, Reduktionsmittel, Alkalisierungsmittel, Antioxidantien, Parfümöle und dergleichen enthalten können.

Die in den Mitteln enthaltenen Tenside, können in den Endzubereitungen gleichfalls als Emulgatoren dienen. Daneben können den Haarfärbepräparaten auch weitere Tenside bzw. **Co-Emulgatoren** zugesetzt werden, wie z.B.:

(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) $C_{12/18}$-Fettsäuremono- und -diester von Anlagerungsprodukten von 1 bis 30 Mol Ethylenoxid an Glycerin;

(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;

(4) Alkylmono- und -oligoglycoside mit 8 bis 22 Kohlenstoffatomen im Alkylrest und deren ethoxylierte Analoga;

(5) Anlagerungsprodukte von 15 bis 60 Mol Ethylenoxid an Ricinusöl und/oder gehärtetes Ricinusöl;

(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;

(7) Anlagerungsprodukte von 2 bis 15 Mol Ethylenoxid an Ricinusöl und/oder gehärtetes Ricinusöl;

(8) Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);

(9) Mono-, Di- und Trialkylphosphate sowie Mono-, Di- und/oder Tri-PEG-alkylphosphate und deren Salze;

(10) Wollwachsalkohole;

(11) Polysiloxan-Polyalkyl-Polyether-Copolymere bzw. entsprechende Derivate;

(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,

(13) Polyalkylenglycole sowie

(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

$C_{8/18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Qligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12/18}$-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976)**.

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher wie in der **EP 0693471 B1** beschrieben;

- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoyl-methan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Seien und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Komplexbildner** können EDTA, NTA, Phosphonsäuren, Triton B, Turpinal und Phenazetin eingesetzt werden. Des weiteren können **Reduktionsmittel**, wie beispielsweise Ascorbinsäure, Natriumsulfat, Natriumthiosulfat und dergleichen enthalten sein. Als **Alkalisierungsmittel** kommen Ammoniak, Monoethanolamine, (L)- Arginin, AMP usw. in Frage.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilliat, Irotyl und Floramat allein oder in Mischungen, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

**Beispiele**

Es wurden Haarfärbepräparate hergestellt, welche die erfindungsgemäßen Mittel 1 und 2 bzw. die Vergleichsmischung V1 enthielten. Die Herstellung der Dispersionen erfolgte nach dem Mikrodispersions-Verfahren. Nach Anwendung dieser Haarfärbecremes wurde zur Untersuchung der Haarstruktur eine thermische Analyse der Humanhaare (Alkinco 6634) durchgeführt. Mit Hilfe der dynamischen Differenz-Kalorimetrie (HP-DSC; F.J. Wortmann et al., J. Appl. Polym. Sci. 1993, 48, S. 137ff.) kann hierbei der Umwandlungspunkt der behandelten Haarprobe im Vergleich zu einer unbehandelten Haarprobe (HP-DSC = 152,5°°C) gemessen werden. Die Stabilität wurde visuell nach Lagerung über einen Zeitraum von 4 Wochen bei 40 °C durch Betrachtung der Trübung (+ = trüb; - = trübungsfrei) bestimmt. Die Ergebnisse sind in Tabelle 1 zusammengefaßt:

Tabelle 1

| **Effekt von O/W-Haarfärbecremes am Haar und Stabilität der Cremes- Mengenangaben als Gew.-% Aktivsubstanz** | | | |
|---|---|---|---|
| **Zusammensetzung / Performance** | **1** | **2** | **3** |
| Laurinsäure-12EO-methylester | 13,5 | 15 | - |
| **Monomuls® 90-O18** Glycerol Oleate | 10 | 10 | - |
| **HC Yellow** | 1,0 | 1,5 | 1,0 |
| **Lanette® O** Cetylstearyl Alcohol | 5 | - | 5 |
| Ammoniak | 2,0 | 2,0 | 2,0 |
| Ammoniumchlorid | 0,5 | 0,5 | 0,5 |
| Tetrasodium EDTA | 0,5 | 0,5 | 0,5 |
| **Dehyquart® E-CA** Hydroxycetyl Hydroxyethyl Dimonium Chloride | 1,0 | 1,0 | 1,0 |
| **Texapon® N70** Sodium Laureth Sulfate | 2,5 | 2,5 | 2,5 |
| Natriumsulfit | 1,0 | 1,0 | 1,0 |
| N,N'-Bis(4-aminophenyl)-piperidin | 5,0 | 5,0 | 5,0 |
| Resorcin | 5,0 | 5,0 | 5,0 |
| Wasser | ad 100 | | |
| ***Stabilität nach 4 W (40 °C)*** | - | - | + |
| **Umwandlungspunkt nach HP-DSC** | 150,7 | 151,1 | 149,8 |

**Patentansprüche**

1. Haarfärbepräparate, enthaltend

   (a) 0,5 bis 25 Gew.-% Fettsäure(polyglycol)ester,
   (b) 0,5 bis 25 Gew.-% Fettsäurepartialglyceride und
   (c) 0,1 bis 15 Gew.-% Haarfarbstoffe

   mit der Maßgabe, daß sich die Mengenangaben mit Wasser und gegebenenfalls weiteren Hilfs- und Zusatzstoffen zu 100 Gew.-% ergänzen.

2. Mittel nach Anspruch 1, **dadurch gekennzeichnet**, daß sie als Komponente (a) Fettsäure(polyglycol)ester der For-

mel **(I)** enthalten,

$$R^1COO(AO)_xR^2 \qquad (I)$$

in der $R^1CO$ für einen linearen oder verzweigten, gesättigten oder ungesättigten Acylrest mit 6 bis 30 Kohlenstoffatomen, x für Zahlen von durchschnittlich 0 bis 30 und AO für einen $CH_2CH_2O$-, $CH_2CH(CH_3)O$- und/oder $CH(CH_3)CH_2O$-Rest und $R^2$ für einen linearen oder verzweigten, gesättigten oder ungesättigten Alkylrest mit 1 bis 4 Kohlenstoffatomen steht.

3. Mittel nach den Ansprüchen 1 und/oder 2, **dadurch gekennzeichnet**, daß sie als Komponente (a) Fettsäure(polyglycol)ester der Formel **(I)** enthalten, in der $R^1CO$ für einen linearen oder verzweigten, gesättigten oder ungesättigten Acylrest mit 8 bis 22 Kohlenstoffatomen, x für Zahlen von durchschnittlich 5 bis 20 und $R^2$ für einen linearen oder verzweigten, gesättigten oder ungesättigten Alkylrest mit 1 und/oder 2 Kohlenstoffatomen steht.

4. Mittel nach mindestens einem der Ansprüche 1 bis 3, **dadurch gekennzeichnet**, daß sie als Komponente (a) Fettsäure(polyglycol)ester der Formel **(I)** enthalten, in der $R^1CO$ für einen linearen oder verzweigten, gesättigten oder ungesättigten Acylrest mit 10 bis 18 Kohlenstoffatomen, x für 10 bis 15 und $R^2$ für Methyl steht.

5. Mittel nach mindestens einem der Ansprüche 1 bis 4, **dadurch gekennzeichnet**, daß sie als Komponente (b) Fettsäurepartialglyceride der Formel **(II)** enthalten,

$$\begin{array}{l} CH_2O(CH_2CH_2O)_aCOR^3 \\ | \\ CHO(CH_2CH_2O)_bR^4 \\ | \\ CH_2O(CH_2CH_2O)_cR^5 \end{array} \qquad (II)$$

in der $R^3CO$ für einen linearen oder verzweigten, gesättigten und/oder ungesättigten Acylrest mit 6 bis 22, vorzugsweise 12 bis 18 Kohlenstoffatomen, $R^4$ und $R^5$ unabhängig voneinander für $R^3CO$ oder OH und die Summe ( a+b+c ) für 0 oder Zahlen von 1 bis 100, vorzugsweise 5 bis 25 steht, mit der Maßgabe, daß mindestens einer der beiden Reste $R^4$ und $R^5$ OH bedeutet.

6. Mittel nach mindestens einem der Ansprüche 1 bis 5, **dadurch gekennzeichnet**, daß sie als Komponente (c) direktziehende Farbstoffe und/oder Oxidationsfarbstoffe enthalten.

7. Mittel nach mindestens einem der Ansprüche 1 bis 6, **dadurch gekennzeichnet**, daß sie weiterhin anionische, nichtionische, kationische und/oder amphotere bzw. zwitterionische Tenside enthalten.

8. Mittel nach mindestens einem der Ansprüche 1 bis 7, **dadurch gekennzeichnet**, daß sie weiterhin Ölkomponenten enthalten, welche ausgewählt sind aus der Gruppe, die gebildet wird von Guerbetalkoholen auf Basis von Fettalkoholen mit 6 bis 18 Kohlenstoffatomen, Estern von linearen $C_6$-$C_{22}$-Fettsäuren mit linearen $C_6$-$C_{22}$-Fettalkoholen, Estern von verzweigten $C_6$-$C_{13}$-Carbonsäuren mit linearen $C_6$-$C_{22}$-Fettalkoholen, Estern von linearen $C_6$-$C_{22}$-Fettsäuren mit verzweigten Alkoholen, Estern von linearen und/ oder verzweigten Fettsäuren mit mehrwertigen Alkoholen und/oder Guerbetalkoholen, Triglyceriden auf Basis $C_6$-$C_{10}$-Fettsäuren, flüssigen Mono-/Di-Triglyceridmischungen auf Basis von $C_6$-$C_{18}$-Fettsäuren, Estern von $C_6$-$C_{22}$-Fettalkoholen und/oder Guerbetalkoholen mit aromatischen Carbonsäuren, Estern von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Ölen, verzweigten primären Alkoholen, substituierten Cyclohexanen, linearen $C_6$-$C_{22}$-Fettalkoholcarbonaten, Guerbetcarbonaten, Estern der Benzoesäure mit linearen und/ oder verzweigten $C_6$-$C_{22}$-Alkoholen, Dialkylethern, Ringöffnungsprodukten von epoxidierten Fettsäureestern mit Polyolen, Siliconölen und/oder aliphatischen bzw. naphthenischen Kohlenwasserstoffen.

**9.** Mittel nach mindestens einem der Ansprüche 1 bis 8, **dadurch gekennzeichnet**, daß sie weiterhin polymere Verdickungsmittel enthalten.

**10.** Verwendung von Mitteln, enthaltend

(a) 0,5 bis 25 Gew.-% Fettsäure(polyglycol)ester,
(b) 0,5 bis 25 Gew.-% Fettsäurepartialglyceride und
(c) 0,1 bis 15 Gew.-% Haarfarbstoffe

mit der Maßgabe, daß sich die Mengenangaben mit Wasser und gegebenenfalls weiteren Hilfs- und Zusatzstoffen zu 100 Gew.-% ergänzen, als Konsistenzgeber zur Kaltherstellung von Haarfärbepräparaten.